# EUROPÄISCHE PATENTANMELDUNG

(11) **EP 2 075 724 A2**
(43) Veröffentlichungstag der Anmeldung: **01.07.2009**
(21) Anmeldenummer: 08400050.4
(22) Anmeldetag: 02.12.2008
(51) Int. Cl.: G06F 19/00

(54) **Verfahren und Vorrichtung zur Bereitstellung von Daten von medizintechnischen Komponenten**

(30) Priorität: 28.12.2007 DE 102007063555
(71) Anmelder: Weinmann Geräte für Medizin GmbH & Co. KG, 22525 Hamburg (DE)
(72) Erfinder: Wedler, Wolfgang, 21147 Hamburg (DE)
(74) Vertreter: Klickow, Hans-Henning

(57) **Zusammenfassung**

Das Verfahren und die Vorrichtung dienen zur Bereitstellung von Daten von medizintechnischen Komponenten unter Verwendung von Tags, beispielsweise von RFID-Transpondern. Durch Auswertung der abgespeicherten komponentenspezifischen Informationen können Komponentenkonfigurationen plausibilisiert und/oder Steuerungs- oder Regelungseinstellungen eines medizintechnischen Gerätes automatisch komponentenspezifisch vorgegeben werden. Der Tag ist zur Kommunikation mit einem Reader geeignet.

## Beschreibung

Die Erfindung betrifft eine Vorrichtung zur Bereitstellung von objektspezifischen Daten einer medizintechnischen Komponente mit Informationen, die im Bereich der medizinischen Komponente auf einem Tag, bestehend aus einem Chip und einer Antenne, gespeichert sind.

Die Erfindung betrifft darüber hinaus ein Verfahren zur Identifizierung von objektspezifischen Daten einer medizinischen Komponente, bei dem Informationen im Bereich der medizinischen Komponente gespeichert sind.

Anwender laufen sehr häufig Gefahr, insbesondere bei ähnlichen medizintechnische Geräten, Teile dieser Geräte mit anderen Systemen zu verwechseln und durch die falsche Konnektierung und dem Gebrauch der Komponenten bei medizintechnischen Geräte dem Patienten einen erheblichen Schaden zuzufügen.

Aufgabe der vorliegenden Erfindung ist es, eine Vorrichtung der einleitend genannten Art derart zu verbessern, daß eine einfache Bereitstellung der Informationen unterstützt wird.

Diese Aufgabe wird erfindungsgemäß dadurch gelöst, daß der Tag zur Kommunikation mit einem Reader geeignet ist.

Weitere Aufgabe der vorliegenden Erfindung ist es, ein Verfahren der einleitend genannten Art derart zu verbessern, daß abgespeicherte Informationen in einfacher Weise genutzt werden können.

Diese Aufgabe wird erfindungsgemäß dadurch gelöst, daß die Informationen über Funkkontakt mit einem Reader kommuniziert werden.

Ein erfindungsgemäßer Tag besteht aus einer Antenne und einem Chip, die auf oder in der medizintechnischen Komponente angebracht werden, sowie aus einem Lesegerät, welches mit den Tags kommuniziert. Dadurch ist es möglich, die Gegenstände berührungslos und ohne Sichtkontakt zu identifizieren, zu registrieren sowie objektspezifische Daten und Informationen auszutauschen und ggf. zu verarbeiten.

Eine Ausführungsform sind RFID-Systems bestehend aus einem beschreibbaren Mikrochip, einer Antenne und einem Lesegerät. RFID steht für "Radio Frequency Identification" und bedeutet kontaktlose Identifizierung von Gegenstände mittels Funkübertragung von Daten.

Am zu identifizierenden medizintechnischen Objekt ist ein Tag angebracht. Der Tag besteht aus einem Chip, auf dem Daten gespeichert sind, und einer kleinen Antenne, welche die Daten übermitteln kann. Ein Lesegerät empfängt diese Daten, kann sie entweder selber verarbeiten oder an einen zentralen Computer weiterleiten. Es ist auch vorgesehen Tags in die medizintechnische Komponente zu integriert.

In einer Ausführungsform ist der Tag als Chipkarte aufgebaut mit einer grossen, flache Antenne, die zwischen zwei PVC-Folien einlaminiert ist.

Ein Einbau von Tags in Oberflächen ist vorgesehen zur Identifizierung von beispielsweise Gerätegehäusen aus Kunststoff oder Metallteilen.

Es ist ebenso ein Einsatz von papierdünnen Etiketten vorgesehen, bei denen die Antenne durch Siebdruck oder Ätztechnik auf eine Plastikfolie aufgebracht ist, welche mit einer Papierschicht laminiert und mit Kleber beschichtet wird.

Passive Tags Tags besitzen keine eingebaute Energiequelle, die sie zum Aussenden eines elektromagnetischen Feldes verwenden könnten; sie beziehen die zum Antworten benötigte Energie vollständig aus dem Feld des Lesegerätes.

Aktive Tags beziehen die zum Senden benötigte Energie aus einer eingebauten Quelle. Dies ist in der Regel eine Batterie, die für mehrere Jahre Betriebsdauer ausgelegt ist. Aktive Tags sind für grössere Auslesedistanzen geeignet.

Erfindungsgemäß Tags können auch mittels einer eingebauten Energiequelle ihr Signal aussenden, jedoch nur nach Aufforderung durch ein Lesegerät. Ausserhalb der Lesezeiten befinden sie sich in einem Ruhezustand und senden kein Signal aus.

Bei den erfindungsgemäßen medizintechnischen Komponenten wird entweder nur ein Bit oder eine Identifizierungsnummer übermittelt. In einer Ausführungsform haben die Tags beschreibbare Speicher und können einfache Kommandos zum Lesen oder Schreiben abarbeiten. Dies ermöglicht das Anwenden von einfachen Datenverschlüsselungen, einer Authentifizierung und das gleichzeitige Auslesen mehrerer Tags. Die Daten auf dem Tag können durch das Lesegerät bearbeitet werden, es können neue Informationen auf den Tag geschrieben werden.

In einer weiteren Ausführungsform weisen die Tags einen Mikroprozessor und ein Chipkartenbetriebssystem auf, welches verschlüsselungsalgorithmen ermöglicht.

Die Lesegeräte können entweder die ausgelesenen Daten selber verarbeiten oder mit einem Computer verbunden sein, auf dem die ausgelesenen Daten überprüft, gespeichert und bearbeitet werden.

Hybride Tags, welche sowohl ein kontaktloses als auch ein kontaktbehaftetes Interface haben, sind ebenso vorgesehen. Erfindungsgemäß können auch Strichcodes oder Barcodes oder Strichcodes mit Tags oder Barcodes mit Tags kombiniert werden.

Erfindungsgemäß sind wiederverwendbare Tags oder Einwegtags vorgesehen. Einweg-Tags werden mittels eines Deaktivators so zerstört oder umprogrammiert, dass sie keine Störung des Feldes mehr verursachen.

Das Lesegerät besteht aus einer Sende- und einer Empfangeseinheit. Die Sendeeinheit strahlt ein elektromagnetisches Feld ab, das anschliessend von der Empfangseinheit empfangen und ausgewertet wird. Alternativ sind auch Handlesegeräte vorgesehen, die eine Sende- und eine Empfangseinheit kombinieren.

Tags verändern das von der Sendeeinheit abgestrahlte elektromagnetische Feld auf eine charakteristische Weise, so dass sie detektiert werden können.

Erfindungsgemäß sind alternativ oder ergänzend verschiedene Frequenzen (Langwellen- bis Mikrowellenbereich) mit Reichweiten zwischen 1cm und 100m vorgesehen.

In einer weiterbildung der Erfindung wird die UWB- (Ultra Wide Band) Technologie verwendet, die mit grossen Frequenzbandbreiten arbeitet, jedoch mit geringen Sendeleistungen auskommt.

Erindungsgemäß ist auch das Item-Ievel Tagging vorgesehen. Hierbei weisen zwei Exemplare der gleichen medizintechnischen Komponente, beispielsweise Schläuche für Beatmungsgeräte oder Masken, verschiedene Identifikationsnummern auf.

Neben der Identifikationsnummer können weitere Daten auf dem Tag gespeichert werden, wie Wartungsinformationen, Kennlinien oder Verfallsdaten.

Das Lesegerat kann mehrere Tags in einem Arbeitsgang auslesen. Bei der Logistik der erfindungsgemäßen medizintechnischen Komponenten müssen diese also nicht einzeln am Lesegerät vorbeigeführt werden.

Ein als Echtheitszertifikat ausgebildeter Tag dient dazu, gefälschte Komponenten zu identifizieren.

In Intensivstationen oder im Notfalleinsatz geht es um Leben und Tod. Hier muß der Helfer stets sofort das benötigte Gerät finden. Dazu ist erfindungsgemäß im Gerät ein Tag in aktiver Form vorgesehen, der die Position des lebenswichtigen Gerätes sofort ermitteln lässt.

Eine Statusabfrage von schwer zugänglichen Bauteilen, beispielsweise zur Abfrage des nächsten Serviceintervalls oder als Prüfung der Identität des Bauteils (Fälschung oder Original), ist erfindungsgemäß von außen mittels eines Readers möglich, dadurch, dass das betreffende Bauteil einen Tag aufweist.

Die erfindungsgemäße Idee beschreibt insbesondere auch eine Vorrichtung, bei der mittels eines im Bereich von medizintechnischen Komponenten angebrachten RFID-Chip oder mittels integrierten RFID-Chips eine verbesserte, vereinfachte und sicherere Anwendung von medizintechnischen Komponenten bei einem medizintechnischen Gerät durchgeführt werden kann.

Insbesondere erfolgt die Applikation von RFID-Transpondern zur einfachen und sicheren Anwendung bei medizintechnischen Komponenten.

Beispielhaft kann die medizintechnische Komponente eine Komponente aus der folgenden Gruppe sein:

Blutdruckmanschette, Patientenventil, Flowsensor, Drucksensor, Beatmungsmaske, Atemmaske, Temperaturelemente/- sensoren, EKG-, EOG-, EMG-, EEG-Elektroden, Photosensoren, spektroskopische Sensoren zur Blutparameterbestimmung, Pulsoximetrie-Sensoren, Pulsspektroskopie-Sensoren, taktile Sensoren (Taster, Schalter, leitfähiger Lack), Atemmaske, Beatmungsmaske, Patienteninterface, Tubus, Beatmungszubehör jeglicher Art, Schlauch, Beatmungsschlauch, Anfeuchter, Drucksensor, Filter, Sensor.

Nachfolgend wird beispielhaft für einen Tag immer auf einen RFID Bezug genommen. Alle aufgeführten Erfindungsvarianten sind aber auch mit jedem anderen Tag realisierbar.

In einer weiteren Ausführungsform der Erfindung identifiziert der RFID die Art der medizintechnischen Komponente.

In einer weiteren Ausführungsform der Erfindung identifiziert der RFID die Seriennummer der medizintechnischen Komponente.

In einer weiteren Ausführungsform der Erfindung identifiziert der RFID verschiedene Parameter der medizintechnischen Komponente.

Dabei kann der Parameter beispielhaft mindestens einer aus der folgenden Gruppe sein: Totraumvolumen, Schlauchdurchmesser, Abmaße, Schlauchlänge, Volumen, Volumina, Compliance, Widerstand, Sensorkennlinie, Leckagekennlinie bei verschiedenen Flows, Gewicht, Material.

In einer weiteren Ausführungsform der Erfindung identifiziert der RFID-Chip sicherheitsbezogene Informationen der medizintechnischen Komponente.

In weiteren Ausführungsformen der Erfindung kann die sicherheitsbezogene Information mindestens eine aus der folgenden Gruppe sein: Vollgesichtsmaske, Nasalmaske, Ausatemsystem vorhanden / nicht vorhanden, Notluftventil vorhanden / nicht vorhanden, Leckageeinheit vorhanden / nicht vorhanden.

Insbesondere kommt der erfindungsgemäß deutlich verbesserte Sicherheitsgedanke zum Tragen, wenn beispielsweise ein Beatmungsgerät erkennt, welcher Maskentyp angeschossen ist. Viele Geräte reagieren auf einen Druckabfall innerhalb eines Patientenschlauchsystems und starten dann automatisch, so dass der Nutzer keine weitere Bedienung machen muss. Dies ist jedoch nicht der Fall, wenn eine Maske mit einem Notatemventil angeschlossen ist. Atmet der Nutzer bei aufgesetzter Maske ein und aus, so wird die Ein- und Ausatmung über das Notluftventil geführt; es werden also keine Druckschwankungen über den Schlauch direkt an einen im Gerät angeordneten Drucksensor weitergeführt und detektiert. Dies ist insbesondere bei Fullface-Masken der Fall.

Wird erfindungsgemäß eine Maske mit einem solchen Notatemventil an das Gerät angeschlossen, so kann das Beatmungsgerät mit seinem Reader den Komponententyp oder sicherheitstechnische Informationen wie das Vorhandensein eines Notatemventils, von der Maske abrufen und diese Autostartfunktion automatisch deaktivieren und/oder den Nutzer auf die Deaktivierung der Autostartfunktion hinweisen.

Auch kann in einem weiteren Fall die Verwendung einer Fullfacemaske und die gleichzeitige Nicht-Verwendung eines Lekkageelementes oder eines Ausatemsystems, oder Peep-Ventils angezeigt werden und die Therapie automatisch durch das Gerät verweigert werden. Dabei kann das Gerät die Informationen der RFID-Chips an den verwendeten medizintechnischen Komponenten Beatmungsschlauch und Fullfacemaske ohne Notatemventil lesen und das Fehlen einer Ausatemmöglichkeit detektieren. So kann verhindert werden, dass der Patient bei angeschlossenem Gerät seine ein- und ausgeatmete Luft erneut einatmet und nach mehreren Beatmungszyklen keine Sauerstoffsättigung des Blutes mehr erreicht.

In einer weiteren Ausführungsform der Erfindung ist daran gedacht, mittels der auf dem RFID-Chip abgespeicherten Information zu einer Maske, zwischen eine Vented- und einer Non-Vented-Maske zu unterschieden, so dass das Gerät das Fehlen eines Ausatemsystems erkennt.

Auch kann in einer weiteren Ausführungsform die Gerätesteuerung eines medizintechnischen Gerätes besser auf die verwendeten Komponenten reagieren. Beispielsweise kann das Gerät besser das Totraumvolumen der verwendeten Komponenten berechnen, da diese Information in dem RFID-Chip abgespeichert ist. So kann in einer anderen Anwendung auf Grund der Informationen auf dem RFID-Chip, beispielsweise in einem Beatmungsschlauch, auf die verwendete Schlauchlänge sowie Beatmung rückgeschlossen und ein Sauerstoffgehalt einer Schlauchsektion besser berechnet werden.

In einer weiteren Ausführungsform der Erfindung kann die Regelung und Steuerung des Gerätes besser Rückschlüsse auf die nach dem Drucksensor anfallenden Widerstände von medizintechnischen Komponenten ziehen. So kann das medizintechnische Gerät besser vom Druck, welcher im Gerät gemessen wird, auf den tatsächlich an dem Patienteninterface anliegenden Druck schließen.

In einer weiteren Ausführungsform der Erfindung kann auch sichergestellt werden, ob bereits verwendete Filter erneut in das medizintechnische Gerät eingesetzt worden sind. Das Gerät verweigert dann seine Funktion und/oder gibt eine Meldung aus, dass der eingesetzte Filter seine Betriebsstundenzahl bereits überschritten hat und nicht mehr eingesetzt werden darf.

Folglich kann eine verbesserte Überwachung von auszutauschenden und/oder wartungsfälligen Komponenten durchgeführt werden.

In einer weiteren Ausführungsform der Erfindung kann auf dem RFID-Chip, welcher im Bereich eines Sensors angeordnet ist, die Sensorkennlinie des Sensors abgespeichert sein, so dass ein leichtes und fehlerfreies Auswechseln verschiedener Sensoren möglich ist. Das Gerät nimmt dann die im RFID-Chip eingespeicherte Kennlinie als Basis für die Signalauswertung.

Auch kann bei der Lagerhaltung die Sicherheit verbessert werden, da der Lieferant und/oder Händler bei einem Ausbuchen aus dem Lager durch die Identifizierung verbessert sicherstellen kann, dass auch die richtige evtl. sicherheitsrelevante medizintechnische Komponente an den Kunden ausgegeben werden kann. Darüber hinaus kann in weiteren Ausführungsformen der Erfindung der Lagerhalter sehen, ob noch genug Teile auf Lager sind, eine vereinfachte Lagerhaltung durchführen, ein automatische Bestellen der Mindestbestandskomponenten ausführen und insbesondere in Kliniken die Lagerhaltung optimieren.

In den Zeichnungen sind Ausführungsbeispiele der Erfindung schematisch dargestellt. Es zeigen:
Fig. 1: Eine Vorrichtung zur Beatmung,
Fig. 2: Eine 3-dimensionale Darstellung einer Maske mit integrierter Induktionsantenne, Elektronikkomponente und Sensor,
Fig. 3: Eine beispielhafte Ankopplung der Transpondereinheit an einem Bestandteil eines Gerätes zur Zuführung von Atemgas.

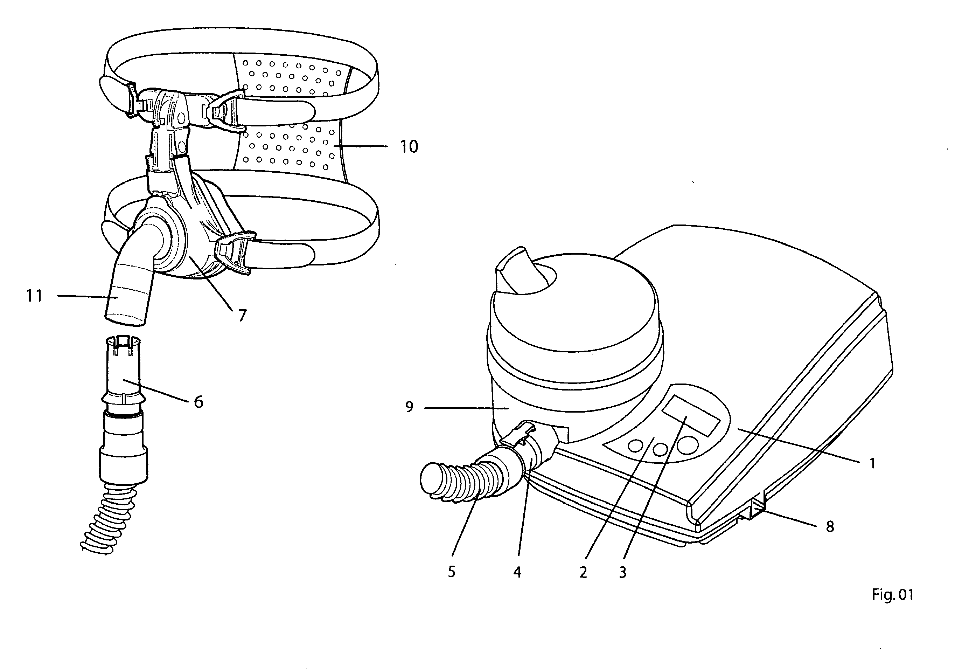

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Zur Ermöglichung einer Datenübertragung weist das Gerätegehäuse (1) eine Schnittstelle (8) auf. Im Bereich einer dem Gerätegehäuse (1) abgewandten Ausdehnung des Verbindungsschlauches (5) ist ein Ausatmungselement (6) angeordnet.

Fig. 1 zeigt darüber hinaus ein Anwender-Interface (7), das als Nasalmaske ausgebildet ist. Eine Fixierung im Bereich eines Kopfes eines Anwenders kann über eine Kopfhaube (10) erfolgen. Im Bereich ihrer dem Verbindungsschlauch (5) zugewandten Ausdehnung weist die Beatmungsmaske (7) einen Anschlussstutzen (11) auf.

Zur Übertragung von Energie durch den Raum ist in das Gerät zur Zuführung von Atemgas ein Reader eingebaut (nicht gezeigt).

Zur Vermeidung eines Austrocknens der Atemwege erweist es sich insbesondere bei längeren Beatmungsphasen als zweckmäßig, eine Befeuchtung der Atemluft durchzuführen. Derartige Befeuchtungen der Atemluft können auch bei anderen Anwendungen realisiert werden. Zur Befeuchtung werden üblicherweise adaptierbare Atemluftbefeuchter (9) in den Luftweg der Vorrichtung zur Zufuhr eines Atemfrischgases zum Nasen-Rachen-Mundraum eines Anwenders eingebracht.

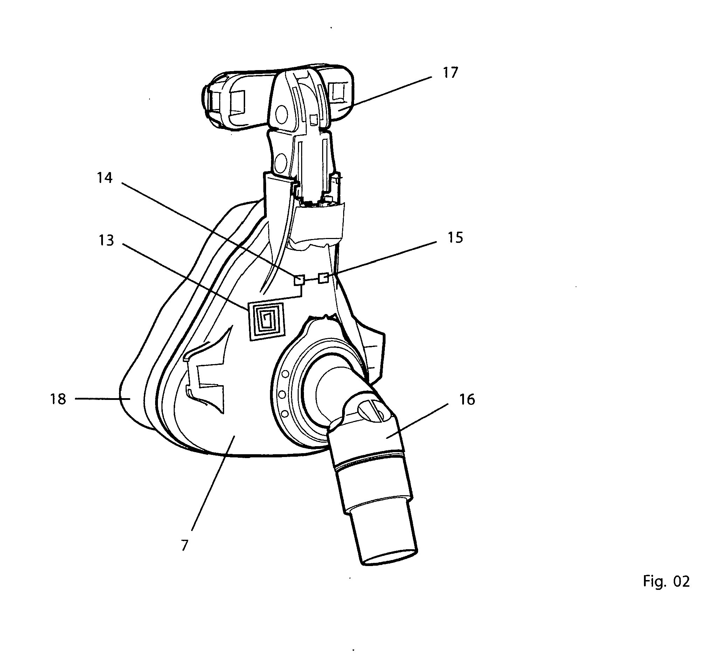

Fig. 2 zeigt einen Aufbau der Erfindung. In einem Teil des Patienteninterfaces (7) bzw. der Patientenmaske, hier ist beispielsweise der Maskengrundkörper gezeigt, befindet sich eine Antenne (13), welche die vom Reader ausgesendete Energie aufnimmt und an eine mit Schaltkreisen versehene Elektronikkomponente (14) weitergibt. Diese erhält Signale von mindestens einem Sensor (15), welcher fest in die Maske integriert sein kann oder als Baustein in die Maske geklickt oder mit einer sonstigen Verbindungstechnik mit der Maske vereinigt werden kann.

Des weiteren können obige Elemente auch in die Stirnstütze (17), den Maskenwulst (18) sowie das Maskenkopplungsstück (16) integriert werden.

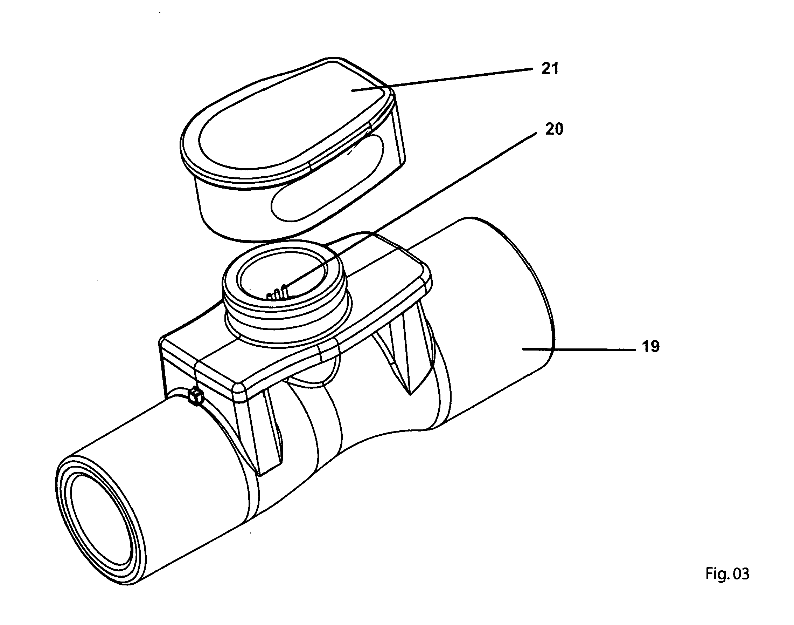

Fig. 3 zeigt eine Ausführungsform, bei der ein Transponder im Bereich eines Zuführelementes (19) angeordnet ist. Das Zuführelement (19) kann mit einer Meßeinrichtung versehen sein, die über Kontakte (20) eine Signalweiterleitung unterstützt und von einer Kappe (21) abdeckbar ist.

## Patentansprüche

1. Vorrichtung zur Bereitstellung von objektspezifischen Daten einer medizintechnischen Komponente mit Informationen, die im Bereich der medizinischen Komponente auf einem Tag, bestehend aus einem Chip und einer Antenne, gespeichert sind, **dadurch gekennzeichnet, dass** der Tag zur Kommunikation mit einem Reader geeignet ist.

2. Vorrichtung nach Anspruch 1, **dadurch gekennzeichnet, daß** auf dem RFID-Chip mindestens Informationen über die medizintechnischen Komponente, insbesondere eine Produktindentifikationsnummer, kodiert sind.

3. Vorrichtung nach Anspruch 1 oder 2, **dadurch gekennzeichnet, daß** die Information ein Parameter der medizintechnischen Komponente ist.

4. Vorrichtung nach einem der Ansprüche 1 oder 2, **dadurch gekennzeichnet, daß** die Information die Identifizierung einer Vollgesichtsmaske ist.

5. Vorrichtung nach einem der Ansprüche 1 oder 2, **dadurch gekennzeichnet, daß** die Information die Identifizierung einer Nasalmaske ist.

6. Vorrichtung nach einem der Ansprüche 1 oder 2, **dadurch gekennzeichnet, daß** die Information "ein Ausatemsystem vorhanden" ist.

7. Vorrichtung nach einem der Ansprüche 1 oder 2, **dadurch gekennzeichnet, daß** die Information "ein Ausatemsystem nicht vorhanden" ist.

8. Vorrichtung nach einem der Ansprüche 1 oder 2, **dadurch gekennzeichnet, daß** die Information "ein Notluftventil vorhanden" ist.

9. Vorrichtung nach einem der Ansprüche 1 oder 2, **dadurch gekennzeichnet, daß** die Information "eine Leckageeinheit vorhanden" ist.

10. Verfahren zur Identifizierung von objektspezifischen Daten einer medizinischen Komponente, bei dem Informationen im Bereich der medizinischen Komponente gespeichert sind, **dadurch gekennzeichnet, daß** die Informationen über Funkkontakt mit einem Reader kommuniziert werden.

11. Verfahren nach Anspruch 10, **dadurch gekennzeichnet, daß** die Information aus dem RFID-Chip für die Steuerung und / oder für die Regelung und / oder für die Kalibrierung eines medizintechnischen Gerätes verwendet wird.

12. Verfahren nach Anspruch 10 oder 11, **dadurch gekennzeichnet, daß** auf Grund der ausgelesenen Information ein Autostart nicht ausführbar ist.

13. Verfahren nach einem der Ansprüche 10 bis 12, **dadurch gekennzeichnet, daß** auf Grund der ausgelesenen Information ein Signal generiert wird.

14. Verfahren nach einem der Ansprüche 10 bis 13, **dadurch gekennzeichnet, daß** durch die aus dem RFID-Chip mindestens einer medizinischen Komponente ausgelesenen Informationen die Steuerung oder die Regelung des medizintechnischen Gerätes derart modifiziert wird, daß mindestens eine Eigenschaft der Komponente bei der Steuerung oder Regelung berücksichtigt wird.

15. Verfahren nach Anspruch 14, **dadurch gekennzeichnet, daß** in Abhängigkeit von den erfaßten Informationen aus abgespeicherten Parametersätzen für Geräteeinstellungen eine bauteilspezifische Voreinstellung aktiviert wird.
